# DEMANDE DE BREVET EUROPEEN

(11) **EP 0 941 725 A1**
(43) Date de publication de la demande: **15.09.1999**
(21) Numéro de dépôt: 99400222.8
(22) Date de dépôt: 02.02.1999
(51) Int. Cl.: A61F 9/06

(54) **Masque de protection pour le soudage à vision dans l'infrarouge et utilisation d'un tel masque**

(30) Priorité: 12.03.1998 FR 9803070
(71) Demandeur: LA SOUDURE AUTOGENE FRANCAISE, F-75321 Paris Cedex 07 (FR)
(72) Inventeur: Briand, Francis, 75010 Paris (FR); Caillibotte, Georges, 78260 Acheres (FR)
(74) Mandataire: Le Moenner, Gabriel

(57) **Abrégé**

Masque (1) de protection utilisable dans une opération de soudage comportant des moyens de protection (6) faciaux, des moyens de maintien (3), des moyens de capture et de transmission (4) de l'image, et des moyens de visualisation (5) de l'image reliés auxdits moyens de capture et de transmission (4) de l'image.

## Description

La présente invention concerne un casque ou masque de soudage permettant notamment une vision dans le proche infrarouge de la scène de soudage.

Habituellement, un masque de soudage comprend des moyens de protection faciaux permettant de protéger le visage ou une partie du visage de l'opérateur d'éventuelles projections de matériaux, lesdits moyens de protection faciaux étant de munis de moyens de maintien permettant de maintenir le masque de soudage en position sur la tête de l'opérateur.

La visualisation de la scène de soudage par l'opérateur se fait au travers d'une fenêtre aménagée au sein des moyens de protection faciaux, laquelle fenêtre est équipée, en général, d'un verre teinté permettant d'atténuer les rayonnements émis par l'arc électrique lors de l'opération de soudage proprement dite.

Il existe plusieurs grades de verres teintés permettant d'obtenir une atténuation plus ou moins importante des rayonnements lumineux; le grade à utiliser dépendant donc de la tâche à accomplir et augmentant avec l'intensité du soudage.

En outre, ce type de masque équipé de verres teintés permet donc à l'opérateur, d'une part, de visualiser l'arc et le bain de soudage et, d'autre part, de protéger ledit opérateur du rayonnement lumineux émis par l'arc électrique, à savoir des rayonnements ultraviolets, infrarouges et/ou visibles.

Il existe également des casques ou masques à cristaux liquides pour lesquels le verre teinté est remplacé par une cassette composée d'une matrice de cristaux liquide prise en sandwich entre deux verres polarisant.

Ces masques présentent notamment l'avantage de permettre un réglage, soit automatique, soit manuel, du niveau d'atténuation désiré.

Ces deux types de masques ne permettent à l'opérateur que d'avoir une vision dans le visible de la scène de soudage, c'est-à-dire des rayonnements visibles émis ou réfléchis par ladite scène de soudage.

Or, dans le spectre visible, l'arc de soudage est la source d'éclairage principale et il s'ensuit que le rayonnement qui en est issu est prépondérant par rapport à toutes les autres sources potentielles d'éclairage.

En d'autres termes, il est difficile de distinguer à travers ces masques de type classique autre chose que l'arc électrique lui-même et que les parties de la scène de soudage éclairée par ledit arc électrique, à savoir essentiellement une partie du bain de soudage et de ses abords immédiat.

Ainsi, il est quasi-impossible de distinguer avec ces masques de l'art antérieur, les zones situées au niveau du bain de soudage mais en dessous de l'arc, ainsi que tout transfert éventuel de matière susceptible de se produire dans l'arc électrique lui-même.

Le but de la présente invention est donc de pallier ces inconvénients des masques de l'art antérieur en proposant un masque de soudage permettant à l'opérateur de discerner en particulier les éléments de la scène de soudage qu'il ne pourrait percevoir avec un masque ordinaire.

La présente invention concerne alors un masque de protection, en particulier pour le soudage ou le coupage, comportant :
- des moyens de protection faciaux,
- des moyens de maintien,
- des moyens de capture et de transmission d'au moins une image, et
- des moyens de visualisation de l'image reliés auxdits moyens de capture et de transmission de l'image.

Il est à noter que dans le cadre de la présente invention les termes "masque de soudage" sont employés dans un sens général, c'est-à-dire qu'ils désignent un masque de protection susceptible d'être mis en oeuvre dans toute opération non seulement de soudage, mais aussi de coupage, de projection thermique et analogue.

De même, on emploie aussi, dans le cadre de l'invention, indifféremment les termes "masque" ou "casque" de protection.

Selon le cas, le masque de la présente invention peut comprendre l'une ou plusieurs des caractéristiques suivantes:
- les moyens de capture et de transmission sont reliés aux moyens de visualisation d'au moins une image par l'intermédiaire de moyens optiques;
- les moyens de visualisation de l'image comprennent au moins un écran vidéo, de préférence au moins un écran à cristaux liquides;
- les moyens de capture et de transmission de l'image comprennent au moins une caméra, de préférence une caméra dont la bande passante est inférieure ou égale à 10 µm, préférentiellement inférieure ou égale à 1100 nm;
- les moyens de capture et de transmission de l'image sont équipés d'au moins un moyen de filtrage, tel un filtre;
- il comporte, en outre, une fenêtre aménagée au sein desdits moyens de protection faciaux, ladite fenêtre étant équipée de moyens à atténuation des rayonnements dans le visible;
- les moyens de visualisation de l'image sont reliés à un système optique de visualisation créant un éloignement suffisant de l'image pour permettre une visualisation efficace de celle-ci par l'opérateur.
- les moyens de visualisation de l'image sont agencés sur ou à l'intérieur du masque de soudage.

L'invention concerne également l'utilisation d'un masque selon l'invention dans un procédé de soudage, de coupage ou de traitement thermique nécessitant l'usage d'un tel masque de soudage, de manière à protéger l'opérateur.

L'invention va maintenant être décrite plus en détail à l'aide de figures données à titre illustratif, mais non limitatif de l'invention.

La figure 1 représente un schéma d'un opérateur 10 muni d'une torche de soudage 11 à l'arc électrique 12, lequel opérateur 10 est équipé d'un masque 1 selon l'art antérieur, c'est-à-dire un masque 1 de soudage muni de moyens de protection faciaux 6 et d'une fenêtre 2 équipée d'un verre teinté permettant d'atténuer une partie du rayonnement émis par l'arc électrique 12.

Les figures 2 à 4 représentent, quant à elles, des masques de soudage selon la présente invention.

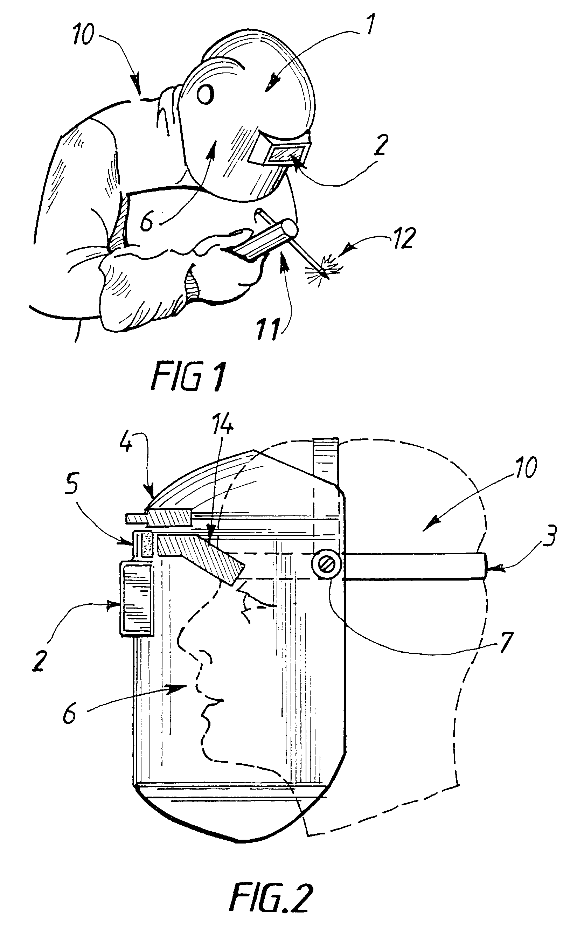

Plus précisément, la figure 2 représente un opérateur 10 équipé d'un masque de soudage 1 selon la présente invention, lequel masque 1 de soudage est équipé de moyens de maintien 3 permettant de maintenir le masque 1 de soudage en position sur la tête de l'utilisateur 10.

Dans ce cas, les moyens de maintien 3 comprennent essentiellement des sangles reliées par des vis, des rivets ou autres moyens analogues 7 aux moyens de protection faciaux 6.

Lesdits moyens de protection faciaux 6 sont constitués principalement d'un écran protecteur 6 en matière plastique par exemple, sur lequel ont été fixé une ou plusieurs caméras 4, par exemple des caméras CCD (Charged Coupling Device) reliées à un ou plusieurs écrans 5, par exemple un ou plusieurs écrans à cristaux liquides ou écrans LCD (Liquid Crystal Display).

Afin de permettre à l'opérateur de visualiser efficacement le ou les écrans 5, il peut être nécessaire, voire indispensable, d'adjoindre aux écrans 5 un système optique 14 de visualisation de l'image, tel par exemple un système de visée analogue à ceux installés sur les caméscopes ou tout système analogue comprenant, de préférence, une ou plusieurs lentilles, et susceptible de permettre un éloignement suffisant de l'image en vue d'une visualisation correcte de celle-ci par l'opérateur.

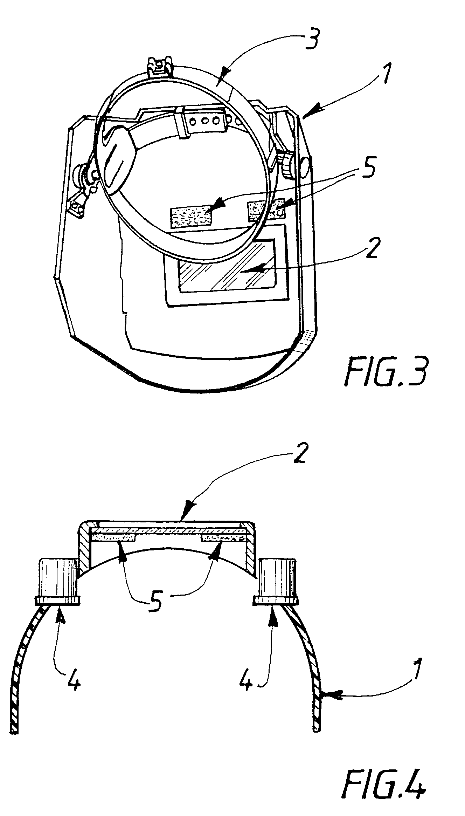

Plus précisément, comme représenté sur les figures 2 et 3, le masque de soudage 1 comporte, dans ce cas, deux écrans LCD agencés du côté interne de masque 1 de soudage au-dessus d'une fenêtre équipée d'un verre teinté 2 de type classique ou une matrice à cristaux liquides.

La figure 4 est un schéma de l'agencement des différentes parties du masque de soudage 1 les unes par rapport aux autres.

Plus précisément, on voit que, dans ce mode de réalisation, le masque de soudage 1 comporte deux caméras CCD 4 placées de part et d'autre et au-dessus de la fenêtre munie d'une vitre teintée 2 ou une matrice à cristaux liquides, lesdites caméras 4 étant reliées à deux écrans 5 à cristaux liquides agencés du côté interne du masque de soudage 1 et au-dessus de la fenêtre à vitre teintée 2, ainsi que représenté sur la figure 3.

Le masque de soudage 1 selon la présente invention permet à l'opérateur 10 de discerner dans la scène de soudage des éléments qu'il ne pourrait percevoir avec un masque classique.

Plus précisément, en capturant l'image de la scène de soudage dans le proche infrarouge, c'est-à-dire entre environ 800 et 1100 nm, on s'affranchit dans une large mesure du rayonnement de l'arc électrique, ce qui permet une visualisation de certaines sources d'éclairage, jusqu'alors inaccessibles, tel notamment le rayonnement issu du métal fondu.

En d'autres termes, le masque de soudage 1 selon la présente invention permet une visualisation plus distinct du bain de soudage et des éventuels transferts de métal entre le fils et le bain de soudage.

Donc pour ce faire, comme expliqué précédemment, on positionne sur le masque de soudage 1 une ou plusieurs caméras 4 ayant une bande passante allant jusqu'à 1 100 nm et équipée, de préférence, d'un filtre permettant d'atténuer le rayonnement visible, par exemple un filtre de type RG1000 commercialisé par la société KODAK.

L'image de la scène de soudage capturée par les caméras 4 est ensuite transférée vers un ou plusieurs écrans de visualisation, tels des écrans à cristaux liquides, lesquels sont agencés sur le masque de soudage 1, de préférence à l'intérieur de celui-ci.

Afin de permettre une visualisation correcte ou adéquat de la scène de soudage par l'opérateur, il peut être avantageux d'équiper les caméras 4 de moyens de zoom et/ou d'autofocus, permettant notamment de conserver une visualisation nette de la scène de soudage lors des mouvements de têtes de l'opérateur.

En l'absence d'objectifs autofocus sur les caméras 4, on prévoit une profondeur de champs de l'objectif suffisamment grande, agrandissement 1 par exemple, afin de conserver, là encore, une image nette lors des mouvements de tête de l'opérateur.

Il est également possible de prévoir sur le masque de soudage selon l'invention, des moyens débrayables à volonté permettant de conserver les fonctionalités des masques classiques, de manière à pouvoir revenir à une visualisation dans le visible de la scène de soudage.

La liaison entre les caméras et les écrans de visualisation se fait par le biais d'une optique adaptée, de manière connue.

Le masque de soudage selon l'invention peut être utilisé dans toute opération de soudage, en particulier à l'arc électrique, nécessitant une visualisation précise et nette de l'ensemble de la scène de soudage.

En particulier, le masque de protection peut être utilisé dans une opération de soudage TIG (Tungsten Inert Gas), MIG (Metal Inert Gas) ou MAG (Metal Active Gas).

## Revendications

1. Masque (1) de protection, en particulier pour le soudage ou le coupage, comportant :
- des moyens de protection (6) faciaux,
- des moyens de maintien (3),
- des moyens de capture et de transmission (4) d'au moins une image, et
- des moyens de visualisation (5) d'au moins une image reliés auxdits moyens de capture et de transmission (4) de l'image.

2. Masque selon la revendication 1, caractérisé en ce que les moyens de capture et de transmission (4) sont reliés aux moyens de visualisation (5) de l'image par l'intermédiaire de moyens optiques.

3. Masque selon la revendication 1, caractérisé en ce que les moyens de visualisation (5) de l'image comprennent au moins un écran "vidéo", de préférence au moins un écran à cristaux liquides.

4. Masque selon la revendication 1, caractérisé en ce que les moyens de capture et de transmission (4) de l'image comprennent au moins une caméra, de préférence une caméra dont la bande passante est inférieure ou égale à 10 µm.

5. Masque selon l'une des revendications 1, 2 ou 4, caractérisé en ce que les moyens de capture et de transmission (4) de l'image sont équipés d'au moins un moyen de filtrage.

6. Masque selon l'une des revendications 1 à 5, caractérisé en ce qu'il comporte, en outre, une fenêtre aménagée au sein desdits moyens de protection (6) faciaux, ladite fenêtre étant équipée de moyens à atténuation (2) des rayonnements dans le visible.

7. Masque selon l'une des revendications 1 à 6, caractérisé en ce que les moyens de visualisation (5) de l'image sont agencés sur ou à l'intérieur du masque de soudage (1).

8. Masque selon l'une des revendications 1 à 7, caractérisé en ce que les moyens de visualisation (5) de l'image sont reliés à un système optique de visualisation (14) créant un éloignement suffisant de l'image pour permettre une visualisation efficace de celle-ci par l'opérateur.

9. Utilisation d'un masque (1) de protection selon l'une des revendications 1 à 8 dans une opération de soudage, de coupage ou de traitement thermique.

10. Utilisation d'un masque (1) de protection selon l'une des revendications 1 à 8 dans une opération de travail à l'arc électrique, en particulier une opération de soudage TIG, MIG ou MAG.
